# EUROPEAN PATENT SPECIFICATION

(11) **EP 1 903 339 B1**
(45) Date of publication and mention of the grant of the patent: **04.08.2010**
(21) Application number: 06019669.8
(22) Date of filing: 20.09.2006
(51) Int. Cl.: G01N 33/68

(54) **Natriuretic peptides and placental-growth factor/soluble VEGF-receptor discriminate cardiac dysfunction related to heart disease from a placenta-associated cardiac dysfunction in pregnant woman**
Natriurethische Peptide und Plazenta-Wachstumfaktor/löslicher VEGF-Rezeptor unterscheiden zwischen mit der Plazenta verbundener Herzdysfunktion und mit Herzerkrankung verbundener Herzdysfunktion
Les peptides natriurétiques et le facteur de croissance placentaire ou le recepteur soluble du VEGF permettent de distinguer entre la dysfonction cardiaque causée par une maladie cardiaque et la dysfonction cardiaque causée par le placenta

(43) Date of publication of application: 26.03.2008
(73) Proprietor: Roche Diagnostics GmbH, 68305 Mannheim (DE); F.HOFFMANN-LA ROCHE AG, 4070 Basel (CH)
(72) Inventor: Hess, Georg, Prof. Dr., 55130 Mainz (DE); Horsch, Andrea, Dr., 68259 Mannheim (DE); Zdunek, Dietmar, Dr., 82327 Tutzing (DE)
(74) Representative: Huhn, Michael

(56) References cited:
- WO-A-03/087819
- WO-A-2006/045593
- US-A1- 2006 008 829
- RESNIK ET AL: "Evaluation of B-type natriuretic peptide (BNP) levels in normal and preeclamptic women" AMERICAN JOURNAL OF OBSTETRICS & GYNECOLOGY, MOSBY, ST LOUIS, MO, US, vol. 193, no. 2, August 2005 (2005-08), pages 450-454, XP005079566 ISSN: 0002-9378
- WU A H B: "MARKERS FOR EARLY DETECTION OF CARDIAC DISEASES" SCANDINAVIAN JOURNAL OF CLINICAL AND LABORATORY INVESTIGATION, BLACKWELL SCIENTIFIC PUBLICATIONS, OXFORD, GB, vol. 65, no. SUPPL 240, 2005, pages 112-121, XP008062995 ISSN: 0085-591X

## Description

The present invention relates to the use of biomarkers for diagnosing a cardiac dysfunction in a pregnant woman, in particular for distinguishing a placenta-associated cardiac dysfunction from cardiac dysfunction related to heart disease.

Preeclampsia is one of the most common disorders of pregnancy, affecting about 5% of pregnancies. It is a major cause of marternal mortality and morbidities, perinatal deaths, preterm birth, and intrauterine growth restriction. Preeclampsia is a syndrome of hypertension, edema, and proteinuria, the symptoms appear after the 20 th week of pregnancy and are usually detected by routine monitoring of the woman's blood pressure and urine. Pre-eclampsia is diagnosed when a pregnant woman develops high blood pressure (two separate readings taken at least 6 hours apart of 140/90 or more) and 300 mg of protein in a 24 hour urine sample (proteinuria). Preeclampsia is also more common in women who have preexisting hypertension, diabetes, or renal disease, in women with a family history of pre-eclampsia, and in women with a multiple gestation (twins, triplets and more).
Key findings support a causal or pathogenic model of superficial placentation driven by immune maladaptation, with subsequently reduced concentrations of angiogenic growth factors and increased placental debris in the maternal circulation resulting in maternal inflammation response. Woman at risk are identified on the basis of epidemiological and clinical risk factors, but the diagnostic criteria of preeclampsia remain unclear.

Pregnancy involves the coordinated formation of new vessel, a process known as angiogenesis. Several growth factors and specific receptors, e.g. vascular endothelial growth factor (VEGF), placental growth factor (P1GF) and soluble Flts-1 (sFlts-1) play essential roles in this process. Preeclampsia is associated with aberrant expression of these molecules, which can be measured in blood samples of pregnant women. VEGF is an endothelial cell-specific mitogen, an angiogenic inducer, and a mediator of vascular permeability. VEGF has been shown to be important for glomerular capillary repair. VEGF binds a homodimer of the membrane-spanning tyrosine kinase-receptor, the fins-like tyrosine kinase (Flt-1), which is differentially expressed in endothelial cells obtained from many different tissues. Flt-1 is highly expressed by trophoblast cells which contribute to placental development. PIGF is expressed by cytotrophoblasts and syncytiotrophoblasts and is capable of inducing proliferation, migration, and activation of endothelial cells. PIGF binds as a homodimer to the Flt-1 receptor. Both PIGF and VEGF contribute to the mitogenic activity and angiogenesis that are critical for the developing placenta. sFlt-1, a splice variant of the Flt-1 receptor, which lacks the transmembrane and cytoplasmic domains of the receptor, binds to VEGF with a high affinity but does not stimulate mitogenesis of endothelial cells. It is expressed in placental tissue as well as in human umbilical vein endothelial cells. sFlt-1 is believed to down-regulate the VEGF signaling pathway.

Several studies have been suggested that women who develop pre-eclampsia are at risk of cardiovascular complications in life. Many risk factors and pathophysiological abnormalities of pre-eclampsia are similar to those of coronary-artery disease. Insulin resistence has been impilcated as a common factor. Microvascular dysfunction, which is associated with insulin resistence, could predispose to both coronary heart disease and preeclampsia.
Beside the placenta induced forms of cardiac dysfunction, also primary cardiac dysfunctions can come into consideration for the cause of cardiac dysfunction of pregnant women. The main cause for primary cardiac dysfunctions are congenitally and aquired heart value diseases as well as myocardic diaseases.

A method for monitoring preeclampsia in pregnant woman by measuring the level of sFlt-1, VEGF or PLGF polypeptide in a sample is disclosed in US 2004/0126828 A1.

The authors stated that sFlt-1 levels are elevated in blood samples taken from preeclamptic women. sFlt-1 binds to VEGF and PIGF with high affinity and blocks the mitogenic and angiogenic activity of these growth factors. The authors suggested that circulating sFlt-1 in patients with preeclampsia may oppose vasorelaxation, thus contributing to hypertension.
Furthermore, US 2005/0025762 A1 disclosed methods for treating preeclampsia and eclampsia by using compounds that decrease sFlt-1 levels, and compounds that inhibit the binding of VEGF or PLGF to sFlt-1. However, cardiac dysfunctions of pregnant women seems to remain undecteted by the solely determination of these angiogenic growth factors.

DE102004051847 disclosed a method for diagnosing atherosclerosis by measuring the level of PIGF and sFlt-1, comprising a method to determine the relationship between PIGF and sFlt-1. Increased levels of PIGF of patients suffering from a myocardiadial infaction is connected with the increased risk for further vascular events. However, the authors predicted that the claimed method only refer to vasculary diseases with atherosclerotic etiology, preeclampsia or eclampsia are excluded from the claimed method.

Furthermore, there have been attemps to determine whether brain natriuretic peptide (BNP) can be used as a biochemical marker in pregnant women suffering from preeclampsia. Resnik et al., (American Journal of Obstetrics and Gynecology (2005) 193, 450 - 4) found that BNP levels are elevated in severe preeclampsia compared to normal pregnancies. The authors presume that ventricular stress and/or subclinical cardial dysfunction is associated with preeclampsia. However, Resnik did not describe if the cardiac dysfunction of the pregnant women are caused by preeclampsia or if the symptoms are due to other preexisting cardiac events.

However, the solely determination of natriuretic peptide does not give evidence for the cause of a cardiac dysfunction in pregnant women, because other reasons like placenta insuffiency were not detected. Thus, in the state of the art there is currently no known diagnostic procedure which allows to differentiate if the cardiac dysfunction in pregnant women is caused by a placenta-associated cardiac dysfunction or if it is caused by a primary heart disease.

Therefore, it is an object of the present invention to provide methods and means for improved diagnosis of cardiac dysfunctions in pregnant women, in particular for the discrimination between a placenta-associated cardiac dysfunction from a primary heart disease.

The present invention relates to a method for diagnosing if a pregnant woman suffers from a cardiac dysfunction related to heart disease or being placenta associated, comprising the steps of a) measuring in vitro in a sample of a pregnant woman, the levels of ai) a Natriuretic peptide in a sample aii) at least one peptide selected from the group Placental-growth Factor and sFlt-1 or a variant thereof comparing the results obtained to reference amounts wherein an increased level of a Natriuretic peptide and a decreased level of Placental-growth Factor and/ or an increased level of sFlt-1 or a variant thereof indicates the presence of a placenta-associated cardiac dysfunction, or wherein an increased level of a Natriuretic peptide and a not decreased level of Placental-growth Factor and/ or a not increased level of sFlt-1 or a variant thereof indicates the presence of a cardiac dysfunction related to heart disease.

In addition the present invention relates to a method for a decision support for the possible treatment of a pregnant woman suffering from a cardiac dysfunction, comprising the steps of a) measuring in vitro in a sample of a pregnant woman the level of ai) a Natriuretic peptide aii) at least one peptide from the group Placental-growth Factor and sFlt-1 or a variant thereof in a sample b) comparing the results obtained to reference amounts c) wherein an increased level of a Natriuretic peptide and a decreased level of Placental-growth Factor and/or an increased level of sFlt-1 or a variant thereof indicates the presence of a placenta-associated cardiac dysfunction, or wherein an increased level of a Natriuretic peptide and a not decreased level of

Placental-Growth Factor and/or an not-increased level of sFlt-1 or a variant thereof indicates the presence of a cardiac dysfunction related to heart disease, d) recommending the initation of the treatment of either a cardiac dysfunction related to heart disease or a placenta-associated cardiac dysfunction, depending on the results obtained in step c).

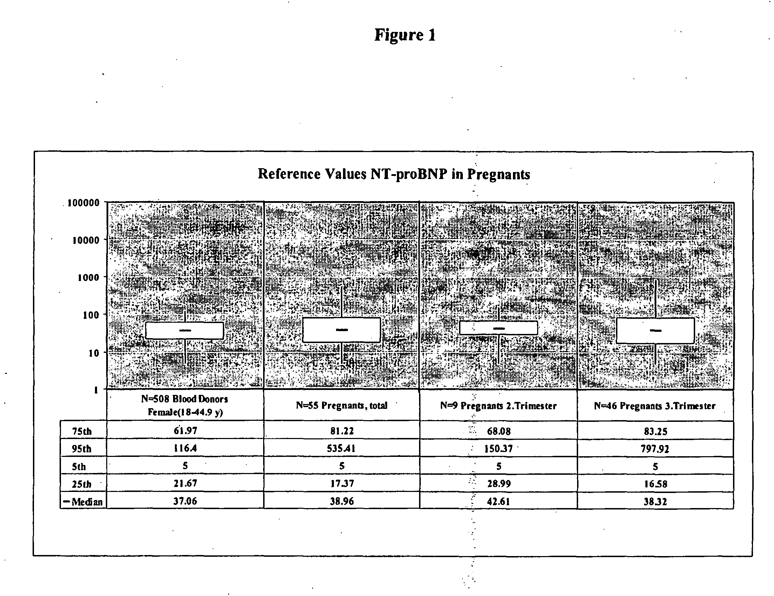

Figure 1 shows box plots for reference values for the NT-proBNP concentration. N represents the number of patients. The first column shows the NT-proBNP concentration of 508 female blood donors from the age of 18 - 44.9 years, who are apparently healthy. This reference valure are compared to the NT-proBNP concentration of 55 pregant women classified in 9 women of the 2. trimester of pregancy and 46 women of the 3. trimester of pregancy. There are no apparently significant differences of NT-proBNP concentration between these groups. Moreover, indicated are the median and the 75^{th}, 95^{th}, and 5^{th} and 25^{th} percentiles.

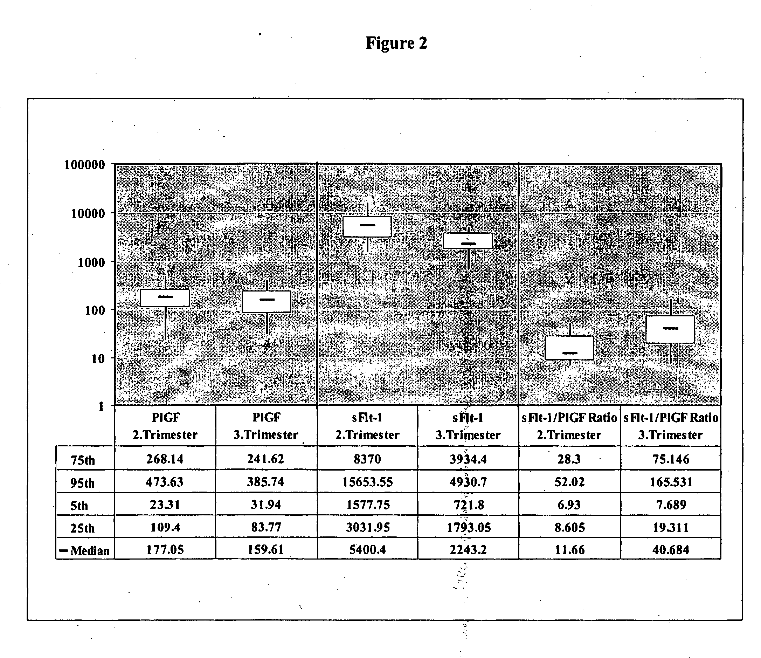

Figure 2 shows box plots for reference values measured for the sFlt-1 concentration and for the PIGF concentration in 46 pregnant women. The NT-proBNP concentration of these 46 pregnant women is less than 125 pg/ml, the group is classified in 14 women of the 2. trimester of pregnancy and 32 women of 3. trimester of prenancy. The concentration of PIGF and sFlt-1 is only slightly decreasing from the 2. to the 3. trimester of pregnancy. Furthermore, a box plot is shown for the sFlt-1/P1GF ratio. The ratio of sFlt-1/P1GF concentration is increasing from the 2. to the 3. trimester of pregnancy.

Moreover, indicated are the median and the 75^{h}, 95^{th}, and 5^{th} and 25^{th} percentiles.

In a first embodiment, the object is achieved by a method for diagnosing in a pregnant woman suffering from a cardiac dysfunction, comprising the steps of
a) measuring the level of a Natriuretic peptide in a sample
b) measuring the level of Placental-growth Factor and/or sFlt-1 or a variant thereof in a sample
wherein an increased level of a Natriuretic peptide and a decreased level of Placental-growth Factor and / or an increased level of sFlt-1 or a variant thereof indicates the presence of a placenta-associated cardiac dysfunction,
or wherein an increased level of a Natriuretic peptide and a not decreased level of Placental-growth Factor and/ or a not increased level of sFlt-1 or a variant thereof indicates the presence of a cardiac dysfunction related to heart disease.

The present method also allows to distinguish a placenta-associated cardiac dysfunction from cardiac dysfunction related to heart disease in a pregnant woman suffering from a cardiac dysfunction.
Analogously, the present invention also relates to a use of the combined information of the measured levels of a natriuretic peptide and Placental-growth Factor and/ or of sFlt-1 for diagnosing a cardiac dysfunction, particularly of a cardiac dysfunction related to a heart disease and/or a placenta-associated cardiac dysfunction, in a pregnant woman presenting with symptoms of a cardiac dysfunction. Such use may be adapted analogously to all other features and preferred embodiments disclosed in the present specification and examples.

A tissue sample according to the present invention refers to any kind of tissue obtained from the dead or alive human or animal body. Tissue samples can be obtained by any method known to the person skilled in the art, for example by biopsy or curettage.

Body fluids according to the present invention may include blood, blood serum, blood plasma, lymphe, cerebral liquor, saliva, vitreous humor, and urine. Particularly, body fluids include blood, blood serum, blood plasma, and urine. Samples of body fluids can be obtained by any method known in the art.

The present invention also provides improved safety in diagnosis of a pregnant woman suffering from a cardiac dysfunction. As laid out above, it has been found in the context of the present invention that an increased level of a Natriuretic peptide and a decreased level of Placental-growth Factor and/ or an increased level of sFlt-1 or a variant thereof indicates the presence of a placenta-associated cardiac dysfunction, wherein an increased level of a Natriuretic peptide and a not decreased level of Placental-growth Factor and/ or a not increased level of sFlt-1 or a variant thereof indicates the presence of a cardiac dysfunction related to heart disease.

It has been found in the context of the present invention that measurement of a natriuretic peptide, in particular of NT-proBNP, alone does not allow to differentiate if the cardiac dysfunction is related to heart disease or to placenta-associated cardiac dysfunction. Combined measurement of a natriuretic peptide and of Placental-growth Factor and/ or of sFlt-1 or a variant thereof may help to avoid false diagnosis, particularly in an emergency setting.

The invention takes advantage of certain "biomarkers" (or simply "markers"), more particularly biochemical or molecular markers. The terms "biomarker", "biochemical marker" and "molecular marker" are known to the person skilled in the art. In particular, biochemical or molecular markers are gene expression products which are differentially expressed (i.e. upregulated or downregulated) in presence or absence of a certain condition, disease, or complication. Usually, a molecular marker is defined as a nucleic acid (such as an mRNA), whereas a biochemical marker is a protein or peptide. The level of a suitable biomarker can indicate the presence or absence of the condition or disease and thus allow diagnosis.

The present invention particularly takes advantage of Placental-growth Factor (PIGF), sFlt-1 and variants thereof and of natriuretic peptides, in particular of NT-proANP and NT-proBNP, as biomarkers, particularly as biochemical markers.

NT-proANP and NT-proBNP belong to the group of natriuretic peptides (see e.g. Bonow, R.O. (1996). New insights into the cardiac natriuretic peptides. Circulation 93: 1946-1950). As already mentioned, NT-proANP and NT-proBNP are generated by proteolytic cleavage from precursor molecules, the pre-pro peptides, resulting in the active hormones (ANP or BNP) and the corresponding N-terminal fragments (NT-proANP and NT-proBNP, respectively).

The pre-pro peptide (134 amino acids in the case of pre-proBNP) comprises a short signal peptide, which is enzymatically cleaved off to release the pro peptide (108 amino acids in the case of proBNP). The pro peptide is further cleaved into an N-terminal pro peptide (NT-pro peptide, 76 amino acids in case of NT-proBNP) and the active hormone (32 amino acids in the case of BNP, 28 amino acids in the case of ANP).

The different cleavage products show several different properties. BNP is produced predominantly (albeit not exclusively) in the ventricles and is released upon increase of wall tension. In contrast, ANP is produced and released exclusively from the atria. ANP and BNP are the active hormones and have a shorter half-life than their respective inactive counterparts, NT-proANP and NT-proBNP. BNP is metabolised in the blood, whereas NT-proBNP circulates in the blood as an intact molecule and as such is eliminated renally. The in-vivo half-life of NT-proBNP is 120 min longer than that of BNP, which is 20 min (Smith MW, Espiner EA, Yandle TG, Charles CJ, Richards AM. Delayed metabolism of human brain natriuretic peptide reflects resistance to neutral endopeptidase. J Endocrinol. 2000; 167: 239-46.).

According to the present invention, the term "a natriuretic peptide" includes ANP and / or BNP or a fragment thereof, and / or NT-proBNP and / or NT-proANP or a variant thereof. The term "a natriuretic peptide" therefore comprises the group consisting of ANP, BNP or a fragment thereof, NT-proBNP, NT-proANP or a variant thereof.
Furthermore, "a natriuretic peptide" includes NT-proBNP or a variant thereof.
A preferred embodiment of the present invention is therefore the measurement of a natriuretic peptide, preferably of ANP and / or a BNP or a fragment thereof, more preferably of NT-proBNP and / or NT-proANP or a variant thereof, most preferably of NT-proBNP or a variant thereof.

Placenta Growth Factor (PIGF, also designated as PGF) is well-known to the person skilled in the art. It is a protein related to the vascular permeability factor (VPF or VEGF). The protein is 149 amino acids long and shares 53% identity with the platelet-derived growth factor-like region of VPF. PIGF appears to be involved in angiogenesis during development, certain periods of adult life, and tumorigenesis.
During early pregnancy, natural killer cells in the uterus accumulate as a dense infiltrate around the invading cytotrophoblast cells. From mid-gestation onwards, these killer cells progressively disappear, which conincides with cytotrophoblast invasion, since human placentation is complete by about 20 weeks' gestation. The uterine natural-killer cells produce several cytokines that are implicated in angiogenesis and vascular stability, including PIGF, VEGF (vascular endothelial growth factor), and angiopoietin 2. In healthy pregnancy, the appropriate interaction between endovascular trophoblast and decidual leucocytes, especially natural-killer cells, results in substantial PIGF and VEGF release. During preeclampsia, the placenta-derived sFlt-1(soluble fms-like tyrosine kinase, also known as soluble VEGF receptor), an antagonist of PIGF and VEGF is upregulated, leading to increased systemic amounts of sFlt-1 that fall after delivery. Raised circulating sFIt-1 in preeclampsia is associated with lowered circulating concentrations of free PIGF and VEGF, resulting in endothelial dysfunction. The magnitude of increase in sFlt-1 correlates with disease severity.

During normal pregnancies, median NT-proBNP-level are not elevated and are stable thoughout gestation. Normal NT-proBNP values of pregnant women correspond to a plasma level of NT-proBNP of less than 125 pg/ml, particularly of less than 76 pg/ml, more particularly of less than 50 pg/ml.

The elevation of NT-proBNP levels of preeclamptic pregnant women is associated with the severity of the disease.
According to the present invention, increased levels of NT-proBNP correspond to a plasma level of NT-proBNP of 125 pg/ml to 300 pg/ml, highly increased levels of NT-proBNP correspond to a plasma level of NT-proBNP of 300 pg/ml to more than 500 pg/ml indicating a cardiac dysfunction relating to a primary heart disease or to placenta-associated cardiac dysfunction.

According to the present invention the term "a not decreased level of PIGf and / or a not increased level of sFlt-1 and / or the sFLt-1/PIGF ratio" refers to levels of control samples of a healthy reference colletive. This reference collective includes samples of healthy pregnant women not suffering from preeclampsia or a primary heart disease.

A "decreased level of PlGf and / or an increased level of sFlt-1 or a modified level of the sFLt-1/PlGF ratio" according to the present invention is indicated if the values differ from a healthy reference colletive, preferably by deviating from the 90^{th} percentile, more preferable by deviating from the 95^{th} percentile, and most preferably by deviating from the 99^{th} percentile.

As it has been found in the context of the present invention that measurement of a natriuretic peptide, in particular of NT-proBNP, alone does not allow to differentiate a cardiac dysfunction related to heart disease from placenta-associated cardiac dysfunction. According to the present invention combined measurement of a natriuretic peptide, Placental-growth Factor and/ or of sFlt-1 or a variant thereof allow to differentiate a cardiac dysfunction related to heart disease from placenta-associated cardiac dysfunction.

The PIGF-levels and sFIt-1 levels during normal pregnancy are not or only slightly decreasing from the 2. to the 3. trimester of pregnancy. These data are shown in Figure 2 of the present invention.
Elevated levels of a Natriuretic peptide and decreasing levels of PIGF and / or increasing levels of sFlt-1 measured during the 2. and 3. trimester of pregnancy indicates the presence of a placenta-associated cardiac dysfunction suffering from preeclampsia. These data are demonstrated in table 1 of the present invention showing that 8 of the 9 pregnant women have PIGF-levels less than 100 pg/ml. Furthermore, these 8 pregnant women have increased levels of NT-proBNP corresponding to a plasma level of NT-proBNP of 125 to 1000 pg/ml.

Elevated levels of a Natriuretic peptide and a not decreased level of PIGF and/ or a not increased level of sFlt-1 or a variant thereof measured during the 2. and 3. trimester of pregnancy indicates the presence of a cardiac dysfunction related to heart disease suffering from a primary heart disease. These datas are demonstrated from patient 92316544 of table 1 of the present invention showing an increased level of NT-proBNP but a normal level of PlGF and sFlt-1.
The term "variants" relates to peptides substantially similar to natriuretic peptides, in particular NT-proANP, NT-proBNP, PIGF, and sFlt-1. The term "substantially similar" is well understood by the person skilled in the art. In particular, a variant may be an isoform or allele which shows amino acid exchanges compared to the amino acid sequence of the most prevalent peptide isoform in the human population. Preferably, such a substantially similar peptide has a sequence similarity to the most prevalent isoform of the peptide of at least 80%, preferably at least 85%, more preferably at least 90%, most preferably at least 95%. Substantially similar are also degradation products, e.g. proteolytic degradation products, which are still recognized by the diagnostic means or by ligands directed against the respective full-length peptide.

The term "variant" also relates to a post-translationally modified peptide such as glycosylated peptide. A "variant" is also a peptide which has been modified after collection of the sample, for example by covalent or non-covalent attachment of a label, particularly a radioactive or fluorescent label, to the peptide. Measuring the level of a peptide modified afer collection of the sample is understood as measuring the level of the originally non-modified peptide.

Examples of particular variants of NT-proANP and NT-proBNP and methods for their measurement are known (Ala-Kopsala, M., Magga, J., Peuhkurinen, K. et al. (2004): Molecular heterogeneity has a major impact on the measurement of circulating N-terminal fragments of A-type and B-type natriuretic peptides. Clinical Chemistry, vol. 50(9), 1576-1588).

The term "diagnosing" is known to the person skilled in the art. Diagnosing is understood as becoming aware of any medical condition, particularly a cardiac disease. Diagnosing also relates to "differential diagnosis", i.e. to distinguishing between different conditions with the same or similar symptoms. Particularly, differential diagnosis includes distinguishing a cardiac dysfunction related to a heart disease from a placenta associated-cardiac dysfunction.

Preferably, the diagnostic information gained by the means and methods according to the present invention is interpreted by a trained physician. Preferably, any decision about further treatment in an individual subject is also made by a trained physician. If deemed appropriate, the physician will also decide about further diagnostic measures.

The term "pregnant woman" according to the present invention preferably relates to a pregnant individual. The individual may have no known history of cardiovascular disease. Preferably, the term "pregnant woman" according to the present invention relates to a pregnant individual showing symptoms of cardiac dysfunction which may be caused by cardiac dysfunction related to a heart disease or which relates to a placenta associated-cardiac dysfunction.

The present invention broadly concerns the diagnosis of cardiac dysfunction in pregnant women. The term "cardiac dysfunction" is known to the person skilled in the art. It relates to any kind of heart dysfunction, more particularly to heart dysfunctions affecting the pumping capability, more particularly it relates to acute and chronic cardiac events.

Patients suffering from a cardiac disease may be individuals suffering from stable angina pectoris (SAP) and individuals with acute coronary syndromes (ACS). ACS patients can show unstable angina pectoris (UAP) or these individuals have already suffered from a myocardial infarction (MI). MI can be an ST-elevated MI or a non-ST-elevated MI. The occurring of an MI can be followed by a left ventricular dysfunction (LVD). Finally, LVD patients undergo congestive heart failure (CHF) with a mortality rate of roughly 15 %. Cardiac diseases according to the present invention also include coronary heart disease, heart valves defects (e.g. mitral valve defects), dilatative cardiomyopathy, hypertroph cardiomyopathy, and heart rhythm defects (arrythmias).

A pregnant woman suffering from a cardiac dysfunction related to heart disease shows an increased level of a Natriuretic peptide and a not decreased level of Placental-growth Factor and/ or a not increased level of sFlt-1 or a variant thereof.
The term "cardiac dysfunction related to heart disease" may relate to the ability of the heart to supply adequate quantities of oxygenated blood to peripheral tissues without adaptation. Cardiac dysfunction can be symptomatic or asymptomatic and can be related to diastolic or systolic dysfunction or both.

A pregnant woman suffering from a cardiac dysfunction related to placenta-associated cardiac dysfunction shows an increased level of a Natriuretic peptide and a decreased level of Placental-growth Factor and/ or an increased level of sFlt-1 or a variant thereof.
The term "placenta-associated cardiac dysfunction" relate to a cardiac dysfunction which has its primary origin in placenta dysfunction and related abnormalities and not primary in the heart.

Symptomatically, cardiac diseases may result in "cardiac insufficiency". The term "cardiac insufficiency" is familiar the person skilled in the art. Preferably, cardiac insuffiency relates to the inability of the heart to circulate the blood sufficiently, particularly under conditions of increased need of oxygenation, such as during physical exercise. Cardiac insufficiency includes both the instability to eject blood sufficiently (forward-failure) as well as the inability to sufficiently take up the venous backflow of blood to the heart (backward-failure).

Cardiac insufficiency may be classified according to a functional classification system established for cardiovascular diseases according to the New York Heart Association (NYHA). Patients of Class I have no obvious symptoms of cardiovascular disease. Physical activity is not limited, and ordinary physical activity does not cause undue fatigue, palpitation, or dyspnea (shortness of breath). Patients of class II have slight limitation of physical activity. They are comfortable at rest, but ordinary physical activity results in fatigue, palpitation, or dyspnea. Patients of class III show a marked limitation of physical activity. They are comfortable at rest, but less than ordinary activity causes fatigue, palpitation, or dyspnea. Patients of class IV are unable to carry out any physical activity without discomfort. They show symptoms of cardiac insufficiency at rest. If any physical activity is undertaken, discomfort is increased.

Another indicator of cardiac insufficiency is the "left ventricular ejection fraction" (LVEF) which is also known as "ejection fraction". People with a healthy heart usually have an unimpaired LVEF, which is generally described as above 50 %. Most people with a systolic cardiac dysfunction which is symptomatic have an LVEF of 40 % or less.

The term "cardiac decompensation" is familiar to the person skilled in the art. "Cardiac decompensation" generally refers to the most severe levels of cardiac insufficiency. During cardiac decompensation, the inability of the heart to circulate the blood sufficiently reaches a level at which the body's stress reactions are unable to compensate for the lack of pumping capacity. Symptoms of cardiac decompensation are known to the person skilled in the art. Particularly, a patient showing symptoms of "cardiac decompensation" is showing symptoms according to NYHA class II, III, IV, or worse. More particularly, the patient shows symptoms according to NYHA class III, IV or worse. Even more particularly, the patient shows symptoms according to NYHA class IV or worse. Most particularly, the patient requires clinical support to stabilize or maintain circulation.

The terms "non-increased" and "increased", and "decreased" level refer to the level of a biomarker measured in a pregnant woman as compared to a known level indicative of the absence of a cardiac dysfunction, particularly in the absence of a cardiac dysfunction related to heart disease or related to placenta-associated cardiac dysfunction.

The person skilled in the art is able to determine known level(s) (or, e.g., ratio(s)). For example, a known level may be determined as the median or the average of the measured levels in a population of individuals not suffering from a cardiac disease. Evaluating the levels in further individuals or patients, e. g. in cohort studies, can help to refine the known levels or ratios. Analogously, it is also possible to define and/or refine reference levels indicative of the presence cardiac dysfunction is related to heart disease or to placenta-associated cardiac dysfunction.

The known level may also be a "reference value". The person skilled in the art is familiar with the concept of reference values (or "normal values") for biomarkers. In particular, the term reference value may relate to the actual value of the level in one or more control samples or it may relate to a value derived from the actual level in one or more control samples. Preferably, samples of at least 2, more preferably at least 5, more preferably at least 50, more preferably at least 100, most preferably at least 500 subjects are analyzed to determine the reference value.

In the most simple case, the reference value is the same as the level measured in the control sample or the average of the levels measured in a multitude of control samples. However, the reference value may also be calculated from more than one control sample. E.g., the reference value may be the arithmetic average of the level in control samples representing the control status (e.g. healthy, particular condition, or particular disease state). Preferably, the reference value relates to a range of values that can be found in a plurality of comparable control samples (control samples representing the same or similar disease status), e.g. the average ± one or more times the standard deviation. Similarly, the reference value may also be calculated by other statistical parameters or methods, for example as a defined percentile of the level found in a plurality of control samples, e.g. a 90 %, 95 %, 97.5%, or 99 % percentile. The choice of a particular reference value may be determined according to the desired sensitivity, specificity or statistical significance (in general, the higher the sensitivity, the lower the specificity and vice versa). Calculation may be carried out according to statistical methods known and deemed appropriate by the person skilled in the art.

The terms "control" or "control sample" are known to the person skilled in the art. Preferably, the "control" relates to an experiment or test carried out to provide a standard, against which experimental results (e.g. the measured level(s) in a patient) can be evaluated. In the present context, the standard preferably relates to the level of the biomarker of interest associated with a particular health or disease status. Thus, a "control" is preferably a sample taken to provide such a standard. E.g., the control sample may be derived from one or more healthy subjects, or from one or more patients representative of a particular disease status.

In the context of the present invention, patients representative of a particular disease status particularly include pregnant women suffering from a cardiac dysfunction related to heart disease or to placenta-associated cardiac dysfunction. All measurements of this group of patients are carried out in the second and / or third trimester of pregnancy. The control sample comprising healthy pregnant women not suffering from preeclampsia are also carried out in the second and / or third trimester of pregnancy. An embodiment of the present invention is therefore, that all measurements are carried out in the second and / or third trimester of pregnancy.

Examples for known levels or ratios are given further below. It will be possible to further refine such levels or ratios. The particular known levels or ratios given in this specification may serve as a guideline for diagnosis. As known and well-accepted in the art, actual diagnosis in the individual subject is preferably carried out through individual analysis by a physician, e.g. depending on weight, age, general health status and anamnesis of the individual subject.

As already mentioned, the underlying cause of a cardiac dysfunction in pregnant women may relate to the presence of a placenta-associated cardiac dysfunction e.g. a pregnant woman suffering from preeclampsia. Furthermore, a pregnant woman may suffer from cardiac dysfunction related to a heart disease e.g. a pregnant woman whose heart function has already been impaired previous to the onset pregnancy.

Therefore, the method according to the present invention may preferably deal with two groups of patients showing symptoms of cardiac dysfunction during pregnancy:
Pregnant women suffering:
   (1) from a primary heart disease,
   (2) from a placenta-associated cardiac dysfunction caused by preeclampsia
In the context of the present invention it was found that pregnant women presenting with symptoms of a primary heart disease (above-mentioned patient group 1) show an increased level of a natriuretic peptide, in particular of NT-proBNP but not decreased level of P1GF and a not increased level of sFlt-1.
Furthermore, in the context of the present invention it was found that patients presenting with symptoms a cardiac dysfunction relating to a placenta- associated cardiac dysfunction (above-mentioned patient group 2) show an increased level of NT-proBNP but a decreased level of PlGF and a increased level of sFlt-1.

According to the present invention, the term "non-increased level of NT-proBNP" preferably corresponds to a plasma level of NT-proBNP of less than 125 pg/ml, particularly of less than 76 pg/ml, more particularly of less than 50 pg/ml.

The elevation of NT-proBNP levels of preeclamptic pregnant women is associated with the severity of the disease.
According to the present invention, increased levels of NT-proBNP correspond to a plasma level of NT-proBNP of 125 pg/ml to 300 pg/ml, highly increased levels of NT-proBNP correspond to a plasma level of NT-proBNP of 300 pg/ml to more than 500 pg/ml.

It is evident that the combined information from Natriuretic peptides and PIGF and/or sFlt-1 may also be expressed differently.
In general, the higher the measured ratio of NT-proBNP to P1GF in a sample of a pregnant woman showing symptoms of a cardiac disease is, the more likely is it that the patient is suffering from placenta-associated cardiac dysfunction caused by preeclampsia.

Furthermore, the person skilled in the art is able to define corresponding levels for samples other than blood plasma.

As can be seen from the examples, measuring the level(s) of PIGF, sFlt-1 and natriuretic peptides, in particular of NT-proANP and NT-proBNP at least one additional time-point may provide additional diagnostic information. For example, measurement of NT-proBNP may help to avoid underestimating the extent of a cardiac disease. Therefore, in a preferred embodiment, the level of PLGF, sFlt-1 and natriuretic peptides, in particular of NT-proANP and NT-proBNP is measured in at least one additional sample, preferably the sample being taken within a short time interval after first measurement. A suitable time may be, for example, within 2 to 12 hours, preferably 4 to 12 hours after taking of the first sample.

In another preferred embodiment, additional diagnostic parameters of cardiac disease are measured, particularly chosen from the group consisting of (a) left ventricular ejection fraction (LVEF), (b) echocardiogram (c) anamnesis (medical history), in particular concerning angina pectoris, (d) electrocardiogram, (e) parameters of thyroid or kidney function, (f) blood pressure, in particular arterial hypertension, (g) thallium scintigram, (h) angiography, (i) catheterization.

These additional diagnostic parameters may be determined before, after, or in parallel to measuring PIGF, sFlt-1 and natriuretic peptides. The additional diagnostic parameters may either establish a suspicion of the presence of a cardiac dysfunction or they may serve to further evaluate the diagnostic relevance of a particular level or ratio measured.

Measurement of PIGF, sFlt-1 and natriuretic peptides may be carried out in parallel or successively. Preferably, measurement is carried out in parallel. The term "parallel" in this context relates to using samples taken at the same time, preferably taken less than 2 hours apart, more preferably taken less than 1 hour apart. Most preferably "parallel" in this context relates to using the same sample. Preferably, also determining the amount or concentration of the peptides in the sample is carried out at the same time.

In another preferred embodiment, additionally at least one biomarker of preeclampsia is measured. Biomarkers for preeclampsia are known to the person skilled in the art. Such markers indicate the presence of preeclampsia in pregnant women. Many placental factors seen in maternal circulation during healthy pregnancy are increased in preeclampsia. These include several inflammatory cytokines, corticotropin-releasing hormone, free-radical species, and activin A, comprising factors stimulating the maternal inflammatory resonse.
Examples for biomarkers of preeclampsia include factors like α2-macroglobulin, CD40 Ligand, Urotensin II and others.
The level of a biochemical or molecular marker can be determined by measuring the concentration of the protein (peptide or polypeptide) or the corresponding transcript. In this context, the term "measuring" relates preferably to a quantitative or semi-quantitative determination of the level.

The level can be measured by measuring the amount or the concentration of the peptide or polypeptide. Preferably, the level is determined as the concentration in a given sample. For the purpose of the invention, it may not be necessary to measure the absolute level. It may be sufficient to measure the relative level compared to the level in an appropriate control. Measurement can also be carried out by measuring derivatives or fragments specific of the peptide or polypeptide of interest, such as specific fragments contained in nucleic acid or protein digests.

Measurement of nucleic acids, particularly mRNA, can be performed according to any method known and considered appropriate by the person skilled in the art.

Examples for measurement of RNA include Northern hybridization, RNAse protection assays, in situ hybridization, and aptamers, e.g. Sephadex-binding RNA ligands (Srisawat, C., Goldstein I.J., and Engelke, D.R. (2001). Sephadex-binding RNA ligands: rapid affinity purification of RNA from complex RNA mixtures. Nucleic Acids Research, vol. 29, no. 2 e4).

Furthermore, RNA can be reversely transcribed to cDNA. Therefore methods for measurement of DNA can be employed for measurement of RNA as well, e.g. Southern hybridization, polymerase chain reaction (PCR), Ligase chain reaction (LCR) (see e.g. Cao, W. (2004) Recent developments in ligase-mediated amplification and detection. Trends in Biotechnology, vol. 22 (1), p. 38-44), RT-PCR, real time RT-PCR, quantitative RT-PCR, and microarray hybridization (see e.g. Frey, B., Brehm, U., and Kübler, G., et al. (2002). Gene expression arrays: highly sensitive detection of expression patterns with improved tools for target amplification. Biochemica, vol. 2, p. 27-29).

Measurement of DNA and RNA may also be performed in solution, e.g. using molecular beacons, peptide nucleic acids (PNA), or locked nucleic acids (LNA) (see e.g. Demidov, V.V. (2003). PNA and LNA throw light on DNA. Trends in Biotechnology, vol. 21 (1), p. 4-6).

Measurement of proteins or protein fragments can be carried out according to any method known for measurement of peptides or polypeptides of interest. The person skilled in the art is able to choose an appropriate method.

The person skilled in the art is familiar with different methods of measuring the level of a peptide or polypeptide. The term "level" relates to amount or concentration of a peptide or polypeptide in the sample.

Measuring can be done directly or indirectly. Indirect measuring includes measuring of cellular responses, bound ligands, labels, or enzymatic reaction products.

Measuring can be done according to any method known in the art, such as cellular assays, enzymatic assays, or assays based on binding of ligands. Typical methods are described in the following.

In one embodiment, the method for measuring the level of a peptide or polypeptide of interest comprises the steps of (a) contacting the peptide or polypeptide with a suitable substrate for an adequate period of time, (b) measuring the amount of product.

In another embodiment, the method for measuring the level of a peptide or polypeptide of interest comprises the steps of (a) contacting the peptide or polypeptide with a specifically binding ligand, (b) (optionally) removing non-bound ligand, (c) measuring the amount of bound ligand.

In another embodiment, the method for measuring the level of a peptide or polypeptide of interest comprises the steps of (a) (optionally) fragmenting the peptides or polypeptides of a sample, (b) (optionally) separating the peptides or polypeptides or fragments thereof according to one or more biochemical or biophysical properties (e.g. according to binding to a solid surface or their run-time in a chromatographic setup), (c) determining the amount of one or more of the peptides, polypeptides, or fragments, (d) determining the identity of one or more of the peptides, polypeptides or fragments of step (c) by mass spectrometry. An overview of mass spectrometric methods is given e.g. by Richard D. Smith (2002). Trends in mass spectrometry instrumentation for proteomics. Trends in Biotechnology, Vol. 20, No. 12 (Suppl.), pp. S3-S7).

Other typical methods for measurement include measuring the amount of a ligand binding specifically to the peptide or polypeptide of interest. Binding according to the present invention includes both covalent and non-covalent binding.

A ligand according to the present invention can be any peptide, polypeptide, nucleic acid, or other substance binding to the peptide or polypeptide of interest. It is well known that peptides or polypeptides, if obtained or purified from the human or animal body, can be modified, e.g. by glycosylation. A suitable ligand according to the present invention may bind the peptide or polypeptide also via such sites.

Preferably, the ligand should bind specifically to the peptide or polypeptide to be measured. "Specific binding" according to the present invention means that the ligand should not bind substantially to ("cross-react" with) another peptide, polypeptide or substance present in the sample investigated. Preferably, the specifically bound protein or isoform should be bound with at least 3 times higher, more preferably at least 10 times higher and even more preferably at least 50 times higher affinity than any other relevant peptide or polypeptide.

Non-specific binding may be tolerable, particularly if the investigated peptide or polypeptide can still be distinguished and measured unequivocally, e.g. by separation according to its size (e.g. by electrophoresis), or by its relatively higher abundance in the sample.

Binding of the ligand can be measured by any method known in the art. Preferably, the method is semi-quantitative or quantitative. Suitable methods are described in the following.

First, binding of a ligand may be measured directly, e.g. by NMR or surface plasmon resonance.

Second, if the ligand also serves as a substrate of an enzymatic activity of the peptide or polypeptide of interest, an enzymatic reaction product may be measured (e.g. the amount of a protease can be measured by measuring the amount of cleaved substrate, e.g. on a Western Blot). For measurement of enzymatic reaction products, preferably the amount of substrate is saturating. The substrate may also be labeled with a detectable lable prior to the reaction. Preferably, the sample is contacted with the substrate for an adequate period of time. An adequate period of time refers to the time necessary for a detectable, preferably measurable amount of product to be produced. Instead of measuring the amount of product, the time necessary for appearance of a given (e.g. detectable) amount of product can be measured.

Third, the ligand may be coupled covalently or non-covalently to a label allowing detection and measurement of the ligand. Labeling may be done by direct or indirect methods. Direct labeling involves coupling of the label directly (covalently or non-covalently) to the ligand. Indirect labeling involves binding (covalently or non-covalently) of a secondary ligand to the first ligand. The secondary ligand should specifically bind to the first ligand. Said secondary ligand may be coupled with a suitable label and/or be the target (receptor) of tertiary ligand binding to the secondary ligand. The use of secondary, tertiary or even higher order ligands is often used to increase the signal. Suitable secondary and higher order ligands may include antibodies, secondary antibodies, and the well-known streptavidin-biotin system (Vector Laboratories, Inc.)

The ligand or substrate may also be "tagged" with one or more tags as known in the art. Such tags may then be targets for higher order ligands. Suitable tags include biotin, digoxygenin, His-Tag, Glutathion-S-Transferase, FLAG, GFP, myc-tag, influenza A virus haemagglutinin (HA), maltose binding protein, and the like. In the case of a peptide or polypeptide, the tag is preferably at the N-terminus and/or C-terminus.

Suitable labels are any labels detectable by an appropriate detection method. Typical labels include gold particles, latex beads, acridan ester, luminol, ruthenium, enzymatically active labels, radioactive labels, magnetic labels ("e.g. magnetic beads", including paramagnetic and superparamagnetic labels), and fluorescent labels.

Enzymatically active labels include e.g. horseradish peroxidase, alkaline phosphatase, beta-Galactosidase, Luciferase, and derivatives thereof. Suitable substrates for detection include di-amino-benzidine (DAB), 3,3'-5,5'-tetramethylbenzidine, NBT-BCIP (4-nitro blue tetrazolium chloride and 5-bromo-4-chloro-3-indolyl-phosphate, available as ready-made stock solution from Roche Diagnostics), CDP-Star^{™} (Amersham Biosciences), ECF^{™} (Amersham Biosciences). A suitable enzyme-substrate combination may result in a colored reaction product, fluorescence or chemoluminescence, which can be measured according to methods known in the art (e.g. using a light-sensitive film or a suitable camera system). As for measuring the enyzmatic reaction, the criteria given above apply analogously.

Typical fluorescent labels include fluorescent proteins (such as GFP and its derivatives), Cy3, Cy5, Texas Red, Fluorescein, and the Alexa dyes (e.g. Alexa 568). Further fluorescent labels are available e.g. from Molcular Probes (Oregon). Also the use of quantum dots as fluorescent labels is contemplated.

Typical radioactive labels include 35S, 125I, 32P, 33P and the like. A radioactive label can be detected by any method known and appropriate, e.g. a light-sensitive film or a phosphor imager.

Suitable measurement methods according the present invention also include precipitation (particularly immunoprecipitation), electrochemiluminescence (electro-generated chemiluminescence), RIA (radioimmunoassay), ELISA (enzyme-linked immunosorbent assay), sandwich enzyme immune tests, electrochemiluminescence sandwich immunoassays (ECLIA), dissociation-enhanced lanthanide fluoro immuno assay (DELFIA), scintillation proximity assay (SPA), turbidimetry, nephelometry, latex-enhanced turbidimetry or nephelometry, solid phase immune tests, and mass spectrometry such as SELDI-TOF, MALDI-TOF, or capillary electrophoresis-mass spectrometry (CE-MS). Further methods known in the art (such as gel electrophoresis, 2D gel electrophoresis, SDS polyacrylamid gel electrophoresis (SDS-PAGE), Western Blotting), can be used alone or in combination with labeling or other dectection methods as described above.

Furthermore, suitable methods include microplate ELISA-based methods, fully-automated or robotic immunoassays (available for example on Elecsys^{™} or Cobas^{™} analyzers), CBA (an enzymatic Cobalt Binding Assay, available for example on Roche-Hitachi^{™} analyzers), and latex agglutination assays (available for example on Roche-Hitachi^{™} analyzers).

Preferred ligands include antibodies, nucleic acids, peptides or polypeptides, and aptamers, e.g. nucleic acid or peptide aptamers. Methods to such ligands are well-known in the art. For example, identification and production of suitable antibodies or aptamers is also offered by commercial suppliers. The person skilled in the art is familiar with methods to develop derivatives of such ligands with higher affinity or specificity. For example, random mutations can be introduced into the nucleic acids, peptides or polypeptides. These derivatives can then be tested for binding according to screening procedures known in the art, e.g. phage display.

The term "antibody" as used herein includes both polyclonal and monoclonal antibodies, as well as fragments thereof, such as Fv, Fab and F(ab)2 fragments that are capable of binding antigen or hapten.

In another preferred embodiment, the ligand, preferably chosen from the group consisting of nucleic acids, peptides, polypeptides, more preferably from the group consisting of nucleic acids, antibodies, or aptamers, is present on an array.

Said array contains at least one additional ligand, which may be directed against a peptide, polypeptide or a nucleic acid of interest. Said additional ligand may also be directed against a peptide, polypeptide or a nucleic acid of no particular interest in the context of the present invention. Preferably, ligands for at least three, preferably at least five, more preferably at least eight peptides or polypeptides of interest in the context of the present invention are contained on the array.

Binding of the ligand on the array may be detected by any known readout or detection method, e.g. methods involving optical (e.g. fluorescent), electrochemical, or magnetic signals, or surface plasmon resonance.

In another preferred embodiment, the present invention relates to the use of a ligand specifically binding to a natriuretic peptide, particularly NT-proBNP or a variant thereof and a ligand for PlGF and/or sFlt-1 or a variant thereof for the manufacture of a diagnostic kit for diagnosing a cardiac disease, particularly for distinguishing a cardiac dysfunction related to heart disease from a placenta-associated cardiac dysfunction in a pregnant woman suffering from a cardiac dysfunction. Additionally, a ligand specifically binding to a biomarker of preeclampsia, may be used for manufacture of such a kit.

According to the present invention, the term "array" refers to a solid-phase or gel-like carrier upon which at least two compounds are attached or bound in one-, two- or three-dimensional arrangement. Such arrays (including "gene chips", "protein chips", antibody arrays and the like) are generally known to the person skilled in the art and typically generated on glass microscope slides, specially coated glass slides such as polycation-, nitrocellulose- or biotin-coated slides, cover slips, and membranes such as, for example, membranes based on nitrocellulose or nylon. The array may include a bound ligand or at least two cells expressing each at least one ligand.

It is also contemplated to use "suspension arrays" as arrays according to the present invention (Nolan JP, Sklar LA. (2002). Suspension array technology: evolution of the flat-array paradigm. Trends Biotechnol. 20(1):9-12). In such suspension arrays, the carrier, e.g. a microbead or microsphere, is present in suspension. The array consists of different microbeads or microspheres, possibly labeled, carrying different ligands.

In another preferred embodiment, the present invention relates to an array containing a ligand specifically binding to a natriuretic peptide, particularly NT-proBNP or a variant thereof and a ligand for PIGF and / or sFlt-1 or a variant thereof for, (a) for measuring the level of a natriuretic peptide thereof in a sample of a pregnant women, and (b) for measuring the level of PIGF and/or sFlt-1 or variants thereof in a sample of a pregnant woman, for in vitro diagnosing of a cardiac disease, particularly for distinguishing a cardiac dysfunction related to a heart disease from a placenta-associated cardiac dysfunction determining a Natriuretic peptide and Placental-growth Factor and/or sFIt-1 or a variant thereof.

The invention further relates to a method of producing arrays as defined above, wherein at least one ligand is bound to the carrier material in addition to other ligands.

Methods of producing such arrays, for example based on solid-phase chemistry and photo-labile protective groups, are generally known (US 5,744,305). Such arrays can also be brought into contact with substances or substance libraries and tested for interaction, for example for binding or change of confirmation. Therefore, arrays comprising a peptide or polypeptide as defined above may be used for identifying ligands binding specifically to said peptides or polypeptides.

Peptides and polypeptides (proteins) can be measured in tissue, cell, and body fluid samples, i.e. preferably in vitro. Preferably, the peptide or polypeptide of interest is measured in a body fluid sample.

Some of the samples, such as urine samples, may only contain degradation products, in particular fragments, of the peptide or polypeptide of interest. However, as laid out above, measurement of the level may still be possible as long as the fragments are specific for the peptide or polypeptide of interest.

If necessary, the samples may be further processed before measurement. For example, nucleic acids, peptides or polypeptides may be purified from the sample according to methods known in the art, including filtration, centrifugation, or extraction methods such as chloroform/phenol extraction.

Furthermore, it is contemplated to use so called point-of-care or lab-on-a-chip devices for obtaining the sample and measuring the peptide or polypeptide of interest. Such devices may be designed analogously to the devices used in blood glucose measurement. Thus, a patient will be able to obtain the sample and measure the peptide or polypeptide of interest without immediate assistance of a trained physician or nurse.

The present invention also discloses a kit comprising (a) a means or device for measuring the level of a natriuretic peptide thereof in a sample of a pregnant women, and (b) a means or device for measuring the level of PIGF and/or sFlt-1 or variants thereof in a sample of a pregnant woman, for in vitro diagnosing of a cardiac disease, particularly for distinguishing a cardiac dysfunction related to a heart disease from a placenta-associated cardiac dysfunction.
Preferably, the means according to (a) is a ligand binding specifically to a natriuretic peptide, and/or the means according to (b) is a ligand binding specifically to PlGF and/or sFlt-1 or variants thereof. Additionally, the kit may comprise a means or device, particularly a specifically binding ligand, for measuring the level of a biomarker of preeclampsia in a sample from a patient.

In an preferred embodiment, the present invention relates to the use of such a kit for in vitro diagnosis of a cardiac disease, particularly for distinguishing a cardiac dysfunction related to a heart disease from a placenta-associated cardiac dysfunction in a pregnant woman presenting with symptoms of a cardiac dysfunction, as defined by claim 14.

A further preferred embodiment of the present invention is an immunological rapid test, characterized in that specific antibodies to a natriuretic peptide, and/or sFlt-1 or variants thereof are used, (a) for measuring the level of a natriuretic peptide or a variant thereof in a sample of a pregnant women, and (b) for measuring the level of PIGF and/or sFlt-1 or variants thereof in a sample of a pregnant woman, for in vitro diagnosing of a cardiac disease, particularly for distinguishing a cardiac dysfunction related to a heart disease from a placenta-associated cardiac dysfunction by determining a Natriuretic peptide and Placental-growth Factor and/or sFlt-1 or a variant thereof.

An "Immunological rapid test" according to the present invention is a rapid test for immunologically detectable substances which have been known for a long time for numerous different parameters, for example from WO 97/06439, EP 0 291 194, US 5,591,645, US 4,861,711, US 5,141,850, US 6,506,612, US 5,458,852, US 5,073,484. In these cases the immunological detection reagents (essentially labelled and unlabelled antibodies or antigens) are usually provided in a dry form on a support which allows the transport of a sample liquid (in particular body fluids such as blood, serum, plasma, urine, saliva etc.) on or in the support. For this purpose the support is preferably capillary active, for example a membrane or a plastic support provided with capillary channels. Among experts they are often referred to as immunochromatographic test strips or test devices.

Since the Elecsys® NT-proBNP test can only be carried out in a central laboratory, it is difficult to rapidly determine NT-proBNP outside the routine times. Hence it would be particularly advantageous for the emergency ward if a rapid test were available which could be carried out directly in the emergency ward outside of routine times. This rapid test should, however, ensure the same reference ranges and cut-offs as the reference method in the central laboratory (Elecsys® NT-proBNP) in order to enable a good comparability of the results independently of the type of test that is actually carried out.

The quantitative determination of human placenta growth factor (PIGF) concentrations in cell culture supernates, serum, plasma and urine can be carried out by using the human PIGF Immunoassay "Quantikine" (Catalog Number DPG00) from R&D Systems. The quantitative determination of human soluble Vascular Endothelial Growth Factor Receptor 1 (sVEGF R1) concentrations can be carried out by using the human soluble VEGF R1/Flt-1 Immunoassay "Quantikine" (Catalog Number DVR100B) from R&D Systems. This rapid test should for PIGF and VEGF R1/Flt-1, however, ensure the same reference ranges and cut-offs as the above cited reference method in order to enable a good comparability of the results independently of the type of test that is actually carried out.

The present invention also relates to a method according to claim 15 for a decision support for the possible treatment of a pregnant woman suffering from a cardiac dysfunction. Once a patient has been diagnosed, it may have consequences for the subsequent treatment. If a method according to the present invention indicates that a cardiac disease is present in the patient then treatment may be initiated or adapted. The level(s) and/or ratio(s) of a natriuretic peptide, particularly NT-proBNP and NT-proANP, PlGF and/or sFlt-1 or a variant thereof in a pregnant woman may be monitored at regular intervals.
Furthermore, the subject may be investigated intensively by further diagnosis according to methods known to the skilled cardiologist, such as described earlier in this specification, e.g. electrocardiography, or echocardiography. Treatment may include any measures which generally are associated with improving or restoring heart function.

Treatment of cardiac dysfunction related to heart disease may be different from treatment of a placenta-associated cardiac dysfunction in a pregnant woman. If a method according to the present invention indicates the presence of cardiac dysfunction related to heart disease then treatment may focus on administration of ACE-inhibitors, diuretica, β-blockers, digoxin and others.

If a method according to the present invention indicates the presence of a placenta-associated cardiac dysfunction in a pregnant woman, then treatment may rather focus on aspirin, steroids, delivery at an early stage with or without cardiac treatment.

More particularly, in a further embodiment, the present invention relates to a method for a decision support for the possible treatment of a pregnant woman suffering from a cardiac dysfunction, comprising the steps of a) measuring in vitro in a sample of a pregnant woman the level of ai) a Natriuretic peptide aii) at least one peptide selected from the group Placental-growth Factor and sFlt-1 or a variant thereof in a sample b) comparing the results obtained to reference amounts c) wherein an increased level of a Natriuretic peptide and a decreased level of Placental-growth Factor and/or an increased level of sFlt-1 or a variant thereof indicates the presence of a placenta-associated cardiac dysfunction, or wherein an increased level of Natriuretic peptides and a not decreased level of Placental-Growth Factor and/or an not-increased level of sFlt-1 or a variant thereof indicates the presence of a cardiac dysfunction related to heart disease, d) recommending the initation of the treatment of either a cardiac dysfunction related to heart disease or a placenta-associated cardiac dysfunction depending on the results obtained in step c).

### Examples

A cohort of 55 pregnant women has been clinically investigated for the presence of a placenta-associated cardiac dysfunction or the presence of a cardiac dysfunction related to heart disease. Reference values for sFlt-1, PIGF, and NT-proBNP in pregnant women (N=55) classified in 2.Trimester (N=9) and 3. Trimester (N=46) were determined. The values for sFlt-1 and PIGF in pregnant women with elevated NT-proBNP values (> 125 pg/ml) are shown in table 1.
Blood samples of the pregnant women have been analyzed by the Elecsys NT-proBNP^{™} assay (Roche Diagnostics) for NT-proBNP concentrations. The concentration of sFlt-1 have been analyzed by using the human soluble VEGF R1/Flt-1 Immunoassay "Quantikine" (Catalog Number DVR100B) from R&D Systems. The quantitative determination of human placenta growth factor (PIGF) concentrations was analyzed by using the human PIGF Immunoassay "Quantikine" (Catalog Number DPG00) from R&D Systems.

**Table 1: sFlt-1 and PIGF Values in Pregnant Women with elevated NT-proBNP Values (> 125 pg/ml)**

| **Pregnant No.** | **Week Pregnancy** | **Trimester** | **sFlt-1 [pg/ml]** | **PIGF [pg/ml]** | **NT-proBNP [pg/ml]** |
|---|---|---|---|---|---|
| 92316544 | 24 | **2** | **2645.7** | **324.31** | **181.85** |
| 6 | 26 -33 | **3** | **1062.1** | **96.80** | **336.94** |
| 17 | 26 -33 | **3** | **7551.6** | **93.01** | **125.12** |
| 1 | 34 - 36 | **3** | **8653.8** | **73.97** | **655.69** |
| 16 | 34 - 36 | **3** | **3285.1** | **21.61** | **184.57** |
| 2 | 26 -33 | **3** | **341.6** | **15.31** | **415.13** |
| 2 | 26 -33 | **3** | **165.9** | **11.99** | **940.15** |
| 5 | 26 -33 | **3** | **164.7** | **11.27** | **324.75** |
| 16 | 26 -33 | **3** | **127.3** | **10.69** | **940.52** |

Table 1 resumes the levels of sFlt-1 and PIGF of pregnant women with elevated NT-proBNP values. 9 out of 55 pregnant women shown in table 1 have elevated levels of NT-proBNP of more than 125 pg/ml. Furthermore, 8 of these 9 pregant women (patient 6, 17, 1, 16, 2, 2, 5, 16) have decreased levels of PIGF indicating the presence of a placenta-associated cardiac dysfunction suffering from preeclampsia. The elevation of NT-proBNP levels of preeclamptic pregnant women is associated with the severity of the disease.

1 of the 9 pregnant women, patient 92316544, shows an increased level of NT-proBNP but a normal level of PIGF indicating the presence of a cardiac dysfunction related to heart disease suffering from a primary cardiac disfunction.

**Table 2: Reference Values NT-proBNP in Apparently Healthy Blood Donors Females (18-44.9 years)**

| | Age 18-44.9 | | |
|---|---|---|---|
| median age | | 33 | |
| | *Total* | Male | Female |
| N | 1323 | *815* | *508* |
| Percentile | | | |
| 0 | *20.00* | 20.00 | 20.00 |
| 2.5 | *20.00* | 20.00 | 20.00 |
| 5 | *20.00* | 20.00 | 20.00 |
| 10 | *20.00* | 20.00 | 20.00 |
| 25 | *20.00* | 20.00 | 21.67 |
| 50 | *20.43* | 20.00 | 37.06 |
| 75 | *39.35* | 25.67 | 61.97 |
| 90 | *70.20* | 41.69 | 98.80 |
| 95 | *97.32* | *62.89* | *116.40* |
| 97.5 | *115.00* | *85.75* | *129.70* |
| 100 | *534.40* | *534.40* | *196.30* |

Table 2 comprises reference values for NT-proBNP of a cohort of 1323 apparently healthy blood donors of the age of 18 - 44,9 years. The median age of the blood donor is 33. Blood samples of 508 women have been analyzed for the NT-proBNP concentration. Indicated are the 0, 2,5, 5, 10, 25, 50, 75, 90, 95, 97,5, and 100 percentiles.

Figure 1 shows box plots for reference values for the NT-proBNP concentration. N represents the number of patients. The first column shows the NT-proBNP concentration of 508 female blood donors from the age of 18 - 44.9 years, who are apparently healthy. This reference valure are compared to the NT-proBNP concentration of 55 pregant women classified in 9 women of the 2. trimester of pregancy and 46 women of the 3. trimester of pregancy. There are no apparently significant differences of NT-proBNP concentration between these groups. Moreover, indicated are the median and the 75^{th}, 95^{th}, and 5^{th} and 25^{th} percentiles.

Figure 2 shows box plots for reference values measured for the sFlt-1 concentration and for the PIGF concentration in 46 pregnant women. The NT-proBNP concentration of these 46 pregnant women is less than 125 pg/ml, the group is classified in 14 women of the 2. trimester of pregnancy and 32 women of 3. trimester of prenancy. The concentration of PIGF and sFlt-1 is only slightly decreasing from the 2. to the 3. trimester of pregnancy. Furthermore, a box plot is shown for the sFlt-1/PlGF ratio. The ratio of sFlt-1/PlGF concentration is increasing from the 2. to the 3. trimester of pregnancy.
Moreover, indicated are the median and the 75^{th}, 95^{th}, and 5^{th} and 25^{th} percentiles.

## Claims

1. A method for diagnosing if a pregnant woman suffers from a cardiac dysfunction related to a heart disease or being placenta associated, comprising the steps of
a) measuring in vitro in a sample of a pregnant woman, the levels of
ai) a Natriuretic peptide
aii) at least one peptide selected from the group Placental-growth Factor (P1GF) and soluble fms-like tyrosine kinase (sFlt-1) or a variant thereof
b) comparing the results obtained to reference amounts,
wherein an increased level of a Natriuretic peptide and a decreased level of Placental-growth Factor and/or an increased level of sFlt-1 or a variant thereof indicates the presence of a placenta-associated cardiac dysfunction,
or wherein an increased level of a Natriuretic peptide and a not decreased level of Placental-growth Factor and/or a not increased level of sFlt-1 or a variant thereof indicates the presence of a cardiac dysfunction related to heart disease.

2. The method according to claim 1, wherein measurement of a Natriuretic peptide and Placental-growth Factor and/or sFlt-1 or a variant thereof are performed in parallel.

3. The method according to any of claims 1 and 2, wherein the natriuretic peptide is a BNP-type peptide or a variant thereof.

4. The method according to any of claims 1 to 3, wherein the natriuretic peptide is NT-proBNP or a variant thereof.

5. The method according to any of claims 1 to 4, wherein the natriuretic peptide is a ANP-type peptide or a variant thereof.

6. The method according to any of claims 1 to 5, wherein the sample is a blood sample, preferable blood plasma or blood serum and/or an urine sample.

7. The method according to any of claims 1 to 6, wherein measurement is carried out in the second or third trimester of pregnancy.

8. The method according to any of claims 1 to 7, wherein an increased level of NT-proBNP corresponds to a plasma level of NT-proBNP of 125 pg/ml to 300 pg/ml, a highly increased level of NT-proBNP corresponds to a plasma level of NT-proBNP of 300 pg/ml to more than 500 pg/ml.

9. The method according to any of claims 1 to 8, wherein the non-increased level of NT-proBNP corresponds to a plasma level of less than 125 pg/ml.

10. The method according to any of claims 1 to 9, wherein the level of a Natriuretic peptide and Placental-growth Factor and sFlt-1 or a variant thereof is measured.

11. The method according to claim 10, wherein the sFlt-1/PIGF-ratio is determined.

12. The method according to any of claims 1 to 11, wherein additional at least one biomarker for preeclampsia is measured, preferably chosen from the group consisting of α2-macroglobulin, CD40 Ligand, Urotensin II and others.

13. Use of a Natriuretic peptide and at least one further peptide from the group P1GF and sFlt for in vitro distinguishing a cardiac dysfunction related to a heart disease from a placenta-associated dysfunction in a pregnant woman.

14. Use of an immunological rapid test comprising a ligand specifically binding to a natriuretic peptide and a ligand for P1GF and/or sFlt-1 or variants thereof as a diagnostic kit for in vitro diagnosing if a cardiac dysfunction in a pregnant woman is related to a heart disease or is a placenta-associated cardiac dysfunction.

15. A method for a decision support for the possible treatment of a pregnant woman suffering from a cardiac dysfunction,
comprising the steps of
a) measuring in vitro in a sample of a pregnant woman the levels of
ai) a Natriuretic peptide
aii) at least one peptide selected from the group of Placental-growth Factor and sFlt-1 or a variant thereof
b) comparing the results obtained to reference amounts,
c) wherein an increased level of a Natriuretic peptide and a decreased level of Placental-growth Factor and/or an increased level of sFlt-1 or a variant thereof indicates the presence of a placenta-associated cardiac dysfunction, or wherein an increased level of Natriuretic peptide and a non-decreased level of Placental-Growth Factor and/or a not-increased level of sFlt-1 or a variant thereof indicates the presence of a cardiac dysfunction related to a heart disease,
d) recommending the initiation of the treatment of either a cardiac dysfunction related to a heart disease or a placenta-associated cardiac dysfunction, depending on the results obtained in step c).

## Patentansprüche

1. Ein Verfahren zur Diagnose, ob eine schwangere Frau an einer kardialen Dysfunktion leidet, die mit einer Herzerkrankung assoziiert ist oder mit der Plazenta in Zusammenhang steht, umfassend die Schritte
a) in vitro-Messung in einer Probe einer schwangeren Frau der Spiegel
ai) eines natriuretischen Peptids
aii) wenigstens eines Peptids ausgewählt aus der Gruppe Placental-growth Factor (P1GF) und soluble FMS like tyrosine kinase (sFLT-1) oder einer
Variante davon
b) Vergleich der erhaltenen Ergebnisse mit Referenzmengen,
wobei ein erhöhter Spiegel eines natriuretischen Peptids und ein erniedrigter Spiegel des Placental-growth Factor und/oder ein erhöhter Spiegel von sFlt-1 oder einer Variante davon die Anwesenheit einer mit der Plazenta assoziierten kardialen Dysfunktion anzeigt,
oder wobei ein erhöhter Spiegel eines natriuretischen Peptids und ein nicht erniedrigter Spiegel des Placental-growth Factor und/oder ein nicht erhöhter Spiegel von sFlt-1 oder einer Variante davon die Anwesenheit einer kardialen Dysfunktion, die mit einer Herzerkrankung in Verbindung steht, anzeigt.

2. Das Verfahren nach Anspruch 1, wobei die Messung des natriuretischen Peptids und des Placental-growth Factor und/oder sFlt-1 oder einer Variante davon parallel durchgeführt werden.

3. Das Verfahren nach einem der Ansprüche 1 und 2, wobei das natriuretische Peptid ein BNP-artiges Peptid oder eine Variante davon ist.

4. Das Verfahren nach einem der Ansprüche 1 bis 3, wobei das natriuretische Peptid NT-proBNP oder eine Variante davon ist.

5. Das Verfahren nach einem der Ansprüche 1 bis 4, wobei das natriuretische Peptid ein ANP-artiges Peptid oder eine Variante davon ist.

6. Das Verfahren nach einem der Ansprüche 1 bis 5, wobei die Probe eine Blutprobe, vorzugsweise Blutplasma oder Blutserum, und/oder eine Urinprobe ist.

7. Das Verfahren nach einem der Ansprüche 1 bis 6, wobei die Messung im zweiten oder dritten Trimester der Schwangerschaft durchgeführt wird.

8. Das Verfahren nach einem der Ansprüche 1 bis 7, wobei ein erhöhter Spiegel von NT-proBNP einem Plasmaspielgel von NT-proBNP von 125 pg/ml bis 300 pg/ml, ein stark erhöhter Spiegel von NT-proBNP einem Plasmaspiegel von NT-proBNP von 300 pg/ml bis mehr als 500 pg/ml entspricht.

9. Das Verfahren nach einem der Ansprüche 1 bis 8, wobei der nicht erhöhte Spiegel von NT-proBNP einem Plasmaspiegel von weniger als 125 pg/ml entspricht.

10. Das Verfahren nach einem der Ansprüche 1 bis 9, wobei der Spiegel des natriuretischen Peptids und von Placental-growth Factor und sFlt-1 oder einer Variante davon gemessen wird.

11. Das Verfahren nach Anspruch 10, wobei das Verhältnis von sFlt-1 zu PlGF bestimmt wird.

12. Das Verfahren nach einem der Ansprüche 1 bis 11 wobei zusätzlich wenigstens ein Biomarker der Präeklampsie bestimmt wird, vorzugsweise ausgewählt aus der Gruppe bestehend aus α2-macroglobulin, CD40 Ligand, Urotensin II und andere.

13. Verwendung eines natriuretischen Peptids und wenigstens eines weiteren Peptids aus der Gruppe PlGF und sFlt für die in vitro-Unterscheidung einer kardialen Dysfunktion, die mit einer Herzerkrankung assoziiert ist, von einer mit der Plazenta in Zusammenhang stehenden Dysfunktion in einer schwangeren Frau.

14. Verwendung eines immunologischen Schnelltests enthaltend einen Liganden, der spezifisch ein natriuretisches Peptid bindet, und einen Liganden für P1GF und/oder sFlt-1 oder Varianten davon als diagnostisches Kit für die in vitro-Diagnose, ob eine kardiale Dysfunktion in einer schwangeren Frau in Beziehung zu einer Herzerkrankung steht oder eine mit der Plazenta assoziierte kardiale Dysfunktion ist.

15. Ein Verfahren zur Unterstützung der Entscheidung über die mögliche Behandlung einer an einer kardialen Dysfunktion leidenden schwangeren Frau, umfassend die Schritte
a) in vitro-Messung in einer Probe der schwangeren Frau der Spiegel
ai) eines natriuretischen Peptids
aii) wenigstens eines Peptids ausgewählt aus der Gruppe von Placental-growth Factor und sFlt-1 oder einer Variante davon
b) Vergleich der erhaltenen Ergebnisse mit Referenzmengen,
c) wobei ein erhöhter Spiegel eines natriuretischen Peptids und ein erniedrigter Spiegel von Placental-growth Factor und/oder ein erhöhter Spiegel von sFlt-1 oder einer Variante davon die Anwesenheit einer mit der Plazenta assoziierten kardialen Dysfunktion anzeigt, oder wobei ein erhöhter Spiegel eines natriuretischen Peptids und ein nicht erniedrigter Spiegel von Placental-growth Factor und/oder ein nicht erhöhter Spiegel von sFlt-1 oder einer Variante davon die Anwesenheit einer mit einer Herzerkrankung in Verbindung stehenden kardialen Dysfunktion anzeigt,
d) Empfehlung der Einleitung der Behandlung entweder der kardialen Dysfunktion, die mit einer Herzerkrankung in Verbindung steht, oder der mit der Plazenta assoziierten kardialen Dysfunktion in Abhängigkeit von den in Schritt c) erhaltenen Ergebnissen.

## Revendications

1. Procédé pour poser un diagnostic quant au fait de savoir si une femme enceinte souffre d'un dysfonctionnement cardiaque lié à une maladie cardiaque ou associée au placenta, comprenant les étapes
a) de mesure in vitro, dans un échantillon d'une femme enceinte, des taux
ai) d'un peptide natriurétique ;
aii) d'au moins un peptide choisi parmi le groupe comprenant le facteur de développement du placenta (PlGF) et la forme soluble du récepteur VEGF de type 1 (sFlt-1) ou un de ses variants ;
b) de comparaison des résultats obtenus à des valeurs de référence,
dans lequel une augmentation du taux d'un peptide natriurétique et une diminution du taux du facteur de développement du placenta et/ou une augmentation du taux du sFlt-1 ou d'un de ses variants indiquent la présence d'un dysfonctionnement cardiaque associé au placenta ;
ou dans lequel une augmentation du taux d'un peptide natriurétique et une absence de diminution du taux du facteur de développement du placenta et/ou une absence d'augmentation du taux du sFlt-1 ou d'un de ses variants indiquent la présence d'un dysfonctionnement cardiaque lié à une maladie cardiaque.

2. Procédé selon la revendication 1, dans lequel la mesure d'un peptide natriurétique et celle du facteur de développement du placenta et/ou du sFlt-1 ou d'un de ses variants sont effectuées en parallèle.

3. Procédé selon l'une quelconque des revendications 1 et 2, dans lequel le peptide natriurétique est un peptide de type BNP ou un de ses variants.

4. Procédé selon l'une quelconque des revendications 1 à 3, dans lequel le peptide natriurétique est le NT-proBNP ou un de ses variants.

5. Procédé selon l'une quelconque des revendications 1 à 4, dans lequel le peptide natriurétique est un peptide de type ANP ou un de ses variants.

6. Procédé selon l'une quelconque des revendications 1 à 5, dans lequel l'échantillon est un échantillon de sang, de préférence du plasma sanguin ou du sérum sanguin et/ou un échantillon d'urine.

7. Procédé selon l'une quelconque des revendications 1 à 6, dans lequel la mesure est effectuée au cours du deuxième ou du troisième trimestre de la grossesse.

8. Procédé selon l'une quelconque des revendications 1 à 7, dans lequel une augmentation du taux de NT-proBNP correspond à un taux plasmatique du NT-proBNP de 125 pg/ml à 300 pg/ml, une forte augmentation du taux de NT-proBNP correspondant à un taux plasmatique du NT-proBNP de 300 pg/ml à plus de 500 pg/ml.

9. Procédé selon l'une quelconque des revendications 1 à 8, dans lequel l'absence d'augmentation du taux du NT-proBNP correspond à un taux plasmatique inférieur à 125 pg/ml.

10. Procédé selon l'une quelconque des revendications 1 à 9, dans lequel on mesure le taux d'un peptide natriurétique et du facteur de développement du placenta et du sFlt-1 ou d'un de ses variants.

11. Procédé selon la revendication 10, dans lequel on détermine le rapport sFlt-1/PlGF.

12. Procédé selon l'une quelconque des revendications 1 à 11, dans lequel on mesure en outre au moins un biomarqueur pour la prééclampsie, de préférence choisi parmi le groupe constitué par l'α2-macroglobuline, le ligand CD40, l'urotensine II et analogues.

13. Utilisation d'un peptide natriurétique et d'au moins un peptide supplémentaire choisi parmi le groupe comprenant le PIGF et le sFlt pour opérer une distinction in vitro entre un dysfonctionnement cardiaque lié à une maladie cardiaque et un dysfonctionnement associé au placenta chez une femme enceinte.

14. Utilisation d'un test immunologique rapide comprenant un ligand se liant de manière spécifique à un peptide natriurétique et un ligand pour le PIGF et/ou le sFlt-1 ou un de ses variants à titre de nécessaire diagnostique pour poser un diagnostic in vitro quant au fait de savoir si un dysfonctionnement cardiaque chez une femme enceinte est lié à une maladie cardiaque ou bien représente un dysfonctionnement cardiaque associé au placenta.

15. Procédé pour une aide à la décision pour le traitement possible d'une femme enceinte souffrant d'un dysfonctionnement cardiaque, comprenant les étapes
a) de mesure in vitro, dans un échantillon d'une femme enceinte, des taux
ai) d'un peptide natriurétique ;
aii) d'au moins un peptide choisi parmi le groupe comprenant le
facteur de développement du placenta et le sFlt-1 ou un de ses variants ;
b) de comparaison des résultats obtenus à des valeurs de référence,
c) dans lequel une augmentation du taux d'un peptide natriurétique et une diminution du taux du facteur de développement du placenta et/ou une augmentation du taux du sFlt-1 ou d'un de ses variants indiquent la présence d'un dysfonctionnement cardiaque associé au placenta ; ou dans lequel une augmentation du taux d'un peptide natriurétique et une absence de diminution du taux du facteur de développement du placenta et/ou une absence d'augmentation du taux du sFlt-1 ou d'un de ses variants indiquent la présence d'un dysfonctionnement cardiaque lié à une maladie cardiaque ;
d) de recommandation d'entamer le traitement, soit d'un dysfonctionnement cardiaque lié à une maladie cardiaque, soit d'un dysfonctionnement cardiaque associé au placenta, en fonction des résultats obtenus à l'étape c).
